# FASCICULE DE BREVET EUROPEEN

(11) **EP 1 126 817 B1**
(45) Date de publication et mention de la délivrance du brevet: **06.06.2007**
(21) Numéro de dépôt: 99950834.4
(22) Date de dépôt: 26.10.1999
(51) Int. Cl.: A61Q 5/00, A61K 8/37

(54) **COMPOSITIONS COSMETIQUES DETERGENTES ET UTILISATION**
KOSMETISCHE WASCHMITTELZUSAMMENSETZUNGEN UND DEREN VERWENDUNG
CLEANSING COSMETIC COMPOSITIONS AND USE

(30) Priorité: 04.11.1998 FR 9813865
(43) Date de publication de la demande: 29.08.2001
(73) Titulaire: L'ORÉAL, 75008 Paris (FR)
(72) Inventeur: MÜLLER, Rainer, D-76344 Leopoldshafen (DE); MELLUL, Myriam, F-94240 L'Hay les Roses (FR)
(74) Mandataire: Le Blainvaux Bellegarde, Françoise
(86) Numéro de dépôt international: PCT/FR1999/002601
(87) Numéro de publication internationale: WO 2000/025735

(56) Documents cités:
- EP-A- 0 715 842
- WO-A-98/32420
- FR-A- 2 740 331

## Description

La présente invention concerne de nouvelles compositions cosmétiques à propriétés améliorées destinées simultanément au nettoyage et au conditionnement des matières kératiniques telles que les cheveux, et comprenant, dans un support aqueux cosmétiquement acceptable, une base lavante constituée de tensioactifs à pouvoir détergent dans laquelle est également présent à titre d'agents conditionneurs au moins un ester d'acide carboxylique en C₄-C₆ et d'alcool en C₁₂-C₂₆. L'invention concerne aussi l'utilisation desdites compositions dans l'application cosmétique susmentionnée.

Pour le nettoyage et/ou le lavage des matières kératiniques telles que les cheveux, l'utilisation de compositions détergentes (telles que les shampooings) à base essentiellement d'agents tensioactifs classiques de type notamment anionique, non ionique et/ou amphotère, mais plus particulièrement de type anionique, est courante. Ces compositions sont appliquées sur cheveux mouillés et la mousse générée par massage ou friction avec les mains permet, après rinçage à l'eau, l'élimination des diverses salissures initialement présentes sur les cheveux.

Ces compositions de base possèdent certes un bon pouvoir lavant, mais les propriétés cosmétiques intrinsèques qui leur sont attachées restent toutefois assez faibles, notamment en raison du fait que le caractère relativement agressif d'un tel traitement de nettoyage peut entraîner à la longue sur la fibre capillaire des dommages plus ou moins marqués liés en particulier à l'élimination progressive des lipides ou protéines contenues dans ou à la surface de cette dernière.

Aussi, pour améliorer les propriétés cosmétiques des compositions capillaires détergentes ci-dessus, et plus particulièrement de celles qui sont appelées à être appliquées sur des cheveux sensibilisés (i.e. des cheveux qui se trouvent abîmés ou fragilisés notamment sous l'action chimique des agents atmosphériques et/ou de traitements capillaires tels que permanentes, teintures ou décolorations), il est maintenant usuel d'introduire dans ces dernières des agents cosmétiques complémentaires dits agents conditionneurs destinés principalement à réparer ou limiter les effets néfastes ou indésirables induits par les différents traitements ou agressions que subissent, de manière plus ou moins répétés, les fibres capillaires. Ces agents conditionneurs peuvent bien entendu également améliorer le comportement cosmétique des cheveux naturels.

Dans ce but, on a déjà proposé d'utiliser des silicones et plus particulièrement des silicones insolubles qui se déposent sur les matières kératiniques lors du rinçage. Les silicones présentent cependant l'inconvénient d'être difficiles à maintenir en dispersion régulière dans le milieu.

On a déjà proposé d'utiliser en tant qu'agent conditionneur des huiles telles que les huiles végétales ou animales ou encore des esters d'acides gras. Cependant, les compositions classiques ont des propriétés détergentes et moussantes non satisfaisantes. De plus les matières kératiniques traitées avec ces compositions présentent le plus souvent un toucher gras rédhibitoire.

La présente invention vise à remédier aux inconvénients cités ci-dessus en proposant des compositions conditionnantes et détergentes, suffisamment moussantes, qui présentent de bonnes propriétés de conditionnement, et notamment de démêlage, de douceur et de brillance sans conférer de caractère gras.

Ainsi, à la suite d'importantes recherches menées sur la question, il a maintenant été trouvé par la Demanderesse, qu'en utilisant une base lavante et au moins un ester liquide d'acide carboxylique ayant de 4 à 6 atomes de carbone et d'alcool ayant de 12 à 26 atomes de carbone, il est possible d'obtenir des compositions détergentes stables présentant d'excellentes propriétés d'usage tel qu'un bon pouvoir lavant intrinsèque et de bonnes propriétés moussantes (abondance,

WO9832420 décrit des compositions nettoyantes non émulsifiées pour la peau comprenant des tensioactifs, au moins un ester d'acides carboxylique solide et au moins une huile conditionnante insoluble, notamment un ester liquide de polyol.

FR2 740 331 décrit des compositions de traitement des cheveux et du cuir chevelu comprenant un ou plusieurs esters d'acide butyrique linéaire et un inducteur d'activité de TG. EP 715 842 décrit des émulsions multiples contenant des huiles. tenue de la mousse, démarrage). De plus les compositions présentent de bonnes propriétés cosmétiques, en particulier la facilité de coiffage, le maintien, la nervosité et le volume des cheveux traités.

Les compositions conformes à l'invention confèrent aux cheveux, après rinçage, un remarquable effet traitant qui se manifeste notamment par une facilité de démêlage, ainsi qu'un apport de volume, de légèreté, de lissage, de douceur et de souplesse sans aucune sensation de gras.

Ainsi, la présente invention a pour objet de nouvelles compositions cosmétiques capillaires détergentes et conditionnantes caractérisée par le fait qu'elles comprennent, dans un milieu aqueux cosmétiquement acceptable, (A) 4 à 50% d'une base lavante et (B) au moins un ester liquide d'acide carboxylique ayant de 4 à 6 atomes de carbone et d'alcool ayant de 12 à 26 atomes de carbone de formule (I) selon la revendication 1.

L'invention a également pour objet l'utilisation en cosmétique des compositions ci-dessus pour le nettoyage et/ou le conditionnement des cheveux.

### A-BASE LAVANTE:

Les compositions conformes à l'invention comprennent nécessairement une base lavante, généralement aqueuse.

Le ou les tensioactifs formant la base lavante peuvent être indifféremment choisis, seuls ou en mélanges, au sein des tensioactifs anioniques, amphotères, non ioniques et cationiques.

Toutefois, selon l'invention, la base lavante comprend de préférence des tensioactifs anioniques ou des mélanges de tensioactifs anioniques et de tensioactifs amphotères ou de tensioactifs non ioniques.

Ainsi, selon l'invention, la base lavante peut représenter de 4 % à 50 % en poids, de préférence de 6 % à 35 % en poids, et encore plus préférentiellement de 8 % à 25 % en poids, du poids total de la composition finale.

Les tensioactifs convenant à la mise en oeuvre de la présente invention sont notamment les suivants :

### (i) Tensioactif(s) anionique(s):

Leur nature ne revêt pas, dans le cadre de la présente invention, de caractère véritablement critique.

Ainsi, à titre d'exemple de tensioactifs anioniques utilisables, seuls ou en mélanges, dans le cadre de la présente invention, on peut citer notamment (liste non limitative) les sels (en particulier sels alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, α-oléfine-sulfonates, paraffine-sulfonates ; les alkylsulfosuccinates, les alkyléthersulfosuccinates, les alkylamidesulfosuccinates; les alkylsulfosuccinamates ; les alkylsulfoacétates ; les alkylétherphosphates; les acylsarcosinates ; les acyliséthionates , les acylglutamates et les N-acyltaurates, le radical alkyle ou acyle de tous ces différents composés comportant de préférence de 12 à 20 atomes de carbone, et le radical aryle désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser des tensioactifs faiblement anioniques, comme les acides d'alkyl D galactoside uroniques et leurs sels ainsi que les acides alkyl (C₆-C₂₄) éther carboxyliques polyoxyalkylénés, les acides alkyl(C₆-C₂₄)aryl éther carboxyliques polyoxyalkylénés les acides alkyl(C₆-C₂₄) amido éther carboxyliques polyoxyalkylénés et leurs sels, en particulier ceux comportant de 2 à 50 groupements oxyde d'éthylène, et leurs mélanges.

Parmi les tensioactifs anioniques, on préfère utiliser selon l'invention les sels d'alkylsulfates et d'alkyléthersulfates et leurs mélanges.

### (ii) Tensioactif(s) non ionique(s) :

Les agents tensioactifs non ioniques sont, eux aussi, des composés bien connus en soi (voir notamment à cet égard "Handbook of Surfactants" par M.R. PORTER, éditions Blackie & Son (Glasgow and London), 1991, pp 116-178) et leur nature ne revêt pas, dans le cadre de la présente invention, de caractère critique. Ainsi, ils peuvent être notamment choisis parmi (liste non limitative) les alcools, les alpha-diols, les alkylphénols ou les acides gras polyéthoxylés, polypropoxylés ou polyglycérolés, ayant une chaîne grasse comportant par exemple 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les amines grasses polyéthoxylées ayant de préférence 2 à 30 moles d'oxyde d'éthylène ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl (C₁₀-C₁₄) amines ou les oxydes de N-acylaminopropylmorpholine. On notera que les alkylpolyglycosides constituent des tensioactifs non ioniques rentrant particulièrement bien dans le cadre de la présente invention.

### (iii) Tensioactif(s) amphotère(s):

Les agents tensioactifs amphotères, dont la nature ne revêt pas dans le cadre de la présente invention de caractère critique, peuvent être notamment (liste non limitative) des dérivés d'amines secondaires ou tertiaires aliphatiques, dans lesquels le radical aliphatique est une chaîne linéaire ou ramifiée comportant 8 à 22 atomes de carbone et contenant au moins un groupe anionique hydrosolubilisant (par exemple carboxylate, sulfonate, sulfate, phosphate ou phosphonate) ; on peut citer encore les alkyl (C₈-C₂₀) bétaïnes, les sulfobétaïnes, les alkyl (C₈-C₂₀) amidoalkyl (C₁-C₆) bétaïnes ou les alkyl (C₈-C₂₀) amidoalkyl (C₁-C₆) sulfobétaïnes.

Parmi les dérivés d'amines, on peut citer les produits commercialisés sous les dénomination MIRANOL^{®}, tels que décrits dans les brevets US-2 528 378 et US-2 781 354 et de structures :

R₂ -CONHCH₂CH₂ -N(R₃)(R₄)(CH₂COO-) (2)

dans laquelle : R₂ désigne un radical alkyle dérivé d'un acide R₂-COOH présent dans l'huile de coprah hydrolysée, un radical heptyle, nonyle ou undécyle, R₃ désigne un groupement bêta-hydroxyéthyle et R₄ un groupement carboxyméthyle ; et

R₂-CONHCH₂CH₂-N(B)(C) (3)

dans laquelle :
B représente -CH₂CH₂OX', C représente -(CH₂)_{z} -Y', avec z = 1 ou 2,
X' désigne le groupement -CH₂CH₂-COOH ou un atome d'hydrogène
Y' désigne -COOH ou le radical -CH₂- CHOH - S03H
R₂ désigne un radical alkyle d'un acide R₉ -COOH présent dans l'huile de coprah ou dans l'huile de lin hydrolysée, un radical alkyle, notamment en C₇, C₉, C₁₁ ou C₁₃, un radical alkyle en C₁₇ et sa forme iso, un radical C₁₇ insaturé.

Ces composés sont classés dans le dictionnaire CTFA, 5ème édition, 1993, sous les dénominations Disodium Cocoamphodiacetate, Disodium Lauroamphodiacetate, Disodium Caprylamphodiacetate, Disodium Capryloamphodiacetate, Disodium Cocoamphodipropionate, Disodium Lauroamphodipropionate, Disodium Caprylamphodipropionate, Disodium Capryloamphodipropionate, Lauroamphodipropionic acid, Cocoamphodipropionic acid.
A titre d'exemple on peut citer le cocoamphodiacetate commercialisé sous la dénomination commerciale MIRANOL^{®} C2M concentré par la société RHODIA CHIMIE.

Dans les compositions conformes à l'invention, on utilise de préférence des mélanges d'agents tensioactifs et en particulier des mélanges d'agents tensioactifs anioniques et d'agents tensioactifs amphotères ou non ioniques. Un mélange particulièrement préféré est un mélange constitué d'au moins un agent tensioactif anionique et d'au moins un agent tensioactif amphotère.

On utilise de préférence un agent tensioactif anionique choisi parmi les alkyl(C₁₂-C₁₄) sulfates de sodium, de triéthanolamine ou d'ammonium, les alkyl (C₁₂-C₁₄)éthersulfates de sodium oxyéthylénés à 2,2 moles d'oxyde d'éthylène, le cocoyl iséthionate de sodium et l'alphaoléfine(C₁₄-C₁₆) sulfonate de sodium et leurs mélange avec :
- soit un agent tensioactif amphotère tel que les dérivés d'amine dénommés disodiumcocoamphodipropionate ou sodiumcocoamphopropionate commercialisés notamment par la société RHODIA CHIMIE sous la dénomination commerciale "MIRANOL^{®} C2M CONC" en solution aqueuse à 38 % de matière active ou sous la dénomination MIRANOL^{®} C32;
- soit un agent tensioactif amphotère de type zwittérionique tel que les alkylbétaïnes en particulier la cocoylbétaïne commercialisée sous la dénomination "DEHYTON^{®} AB 30" en solution aqueuse à 32 % de MA par la société HENKEL ou les alkylamidoalkylbétaïnes telles que la TEGOBETAINE^{®} F50 commercialisée par la société GOLDSCHMIDT.

### (iv) Tensioactifs cationiques:

Parmi les tensioactifs cationiques on peut citer en particulier (liste non limitative) les sels d'amines grasses primaires, secondaires ou tertiaires, éventuellement polyoxyalkylénées; les sels d'ammonium quaternaire tels que les chlorures ou les bromures de tétraalkylammonium, d'alkylamidoalkyltrialkylammonium, de trialkylbenzylammonium, de trialkylhydroxyalkyl-ammonium ou d'alkylpyridinium; les dérivés d'imidazoline ; ou les oxydes d'amines à caractère cationique.
On notera que les tensioactifs cationiques, dont l'utilisation n'est pas exclue, ne constituent pas des tensioactifs préférés pour la mise en oeuvre de la présente invention.

### B- ESTERS LIQUIDES

Les compositions selon l'invention comprennent nécessairement au moins un ester liquide d'acide carboxylique ayant de 4 à 6 atomes de carbone et d'alcool ayant de 12 à 26 atomes de carbone. Ces esters sont insolubles dans l'eau à une concentration supérieure à 0.1% à 25°C. Ils sont liquides à température ambiante inférieure à 30°C.

Les esters liquides selon l'invention ont de préférence la formule suivante:

R₁ COOR₂ (I)

dans laquelle :
R₁ désigne un radical hydrocarboné, ou ramifié, éventuellement mono ou polyhydroxylé, ayant de 3 à 5 atomes de carbone.
R₂ désigne un radical hydrocarboné, linéaire ou ramifié, éventuellement mono ou polyhydroxylé, ayant de 12 à 26 atomes de carbone, de préférence ayant de 16 à 22 atomes de carbone,
R₁ désigne de préférence un radical alkyle ramifié ayant de 3 à 5 atomes de carbone, et plus particulièrement un radical tertio-butyle.
R₂ désigne de préférence un radical alkyle saturé ou insaturé ayant 12 à 26 atomes de carbone, plus particulièrement ramifié et encore plus particulièrement choisi parmi les radicaux tridécyle, isocétyle, isostéaryle, octyldodécyle et isoarachidyle.

Les esters liquides particulièrement préférés sont le néopentanoate d'isostéaryle (formule (I) dans laquelle R₁=tertio-butyle et R₂=isostéaryle), le néopentanoate de tridécyle, le néopentanoate d'isocétyle, et le néopentanoate d'isoarachidyle.

Le ou les esters liquides définis ci-dessus peuvent être utilisées dans les compositions conformes à l'invention dans des concentrations généralement comprises entre 0,1 et 20 %, et de préférence entre 0,2 et 10 % en poids par rapport au poids total de la composition et encore plus particulièrement de 0,5 à 5% en poids.

Le milieu aqueux cosmétiquement acceptable peut être constitué uniquement par de l'eau ou par un mélange d'eau et d'un solvant cosmétiquement acceptable tel qu'un alcool inférieur en C₁-C₄, comme l'éthanol, l'isopropanol, le tertiobutanol, le n-butanol ; les alkylèneglycols comme le propylèneglycol, les éthers de glycols.

Les compositions détergentes selon l'invention présentent un pH final généralement compris entre 3 et 10. De préférence, ce pH est compris entre 5 et 8. L'ajustement du pH à la valeur désirée peut se faire classiquement par ajout d'une base (organique ou minérale) dans la composition, par exemple de l'ammoniaque ou une (poly)amine primaire, secondaire ou tertiaire comme la monoéthanolamine, la diéthanolamine, la triéthanolamine, l'isopropanolamine ou la propanediamine-1,3, ou encore par ajout d'un acide minéral ou organique, de préférence un acide carboxylique tel que par exemple l'acide citrique.

Les compositions conformes à l'invention peuvent contenir en plus de l'association définie ci-dessus des agents régulateurs de viscosité tels que des électrolytes, ou des agents épaississants. On peut citer en particulier le chlorure de sodium, le xylène sulfonate de sodium, les scléroglucanes, les gommes de xanthane, les alcanolamides d'acide gras, les alcanolamides d'acide alkyl éther carboxylique éventuellement oxyéthylénés avec jusqu'à 5 moles d'oxyde d'éthylène tel que le produit commercialisé sous la dénomination "AMINOL A15" par la société CHEM Y, les acides polyacryliques réticulés et les copolymères acide acrylique / acrylates d'alkyle en C₁₀-C₃₀ réticulés. Ces agents régulateurs de viscosité sont utilisés dans les compositions selon l'invention dans des proportions pouvant aller jusqu'à 10 % en poids par rapport au poids total de la composition.

Les compositions conformes à l'invention peuvent également contenir jusqu'à 5 % d'agents nacrants ou opacifiants bien connus dans l'état de la technique tels que par exemple les alcools gras supérieurs à C₁₆, les palmitates de sodium ou de magnésium, les stéarates et hydroxystéarates de sodium ou de magnésium, les dérivés acylés à chaîne grasse tels que les monostéarates ou distéarates d'éthylène glycol ou de polyéthylèneglycol, les éthers à chaînes grasses tels que par exemple le distéaryléther ou le 1-(hexadécyloxy)-2-octadécanol.

Les compositions conformes à l'invention peuvent éventuellement contenir en outre d'autres agents ayant pour effet d'améliorer les propriétés cosmétiques de cheveux ou de la peau sans cependant altérer la stabilité des compositions. On peut citer à ce sujet les agents tensioactifs cationiques, les polymères anioniques ou non ioniques ou cationiques ou amphotères, les protéines, les hydrolysats de protéines, les céramides, les pseudocéramides, les acides gras à chaînes linéaires ou ramifiées en C₁₆-C₄₀ tels que l'acide méthyl-18 eicosanoique, les hydroxyacides, les vitamines, le panthénol, les silicones, volatiles ou non volatiles, solubles et insolubles dans le milieu, les huiles végétales, les huiles de synthèses et leurs mélanges.

Les agents conditionneurs de type polymères cationiques utilisables conformément à la présente invention peuvent être choisis parmi tous ceux déjà connus en soi comme améliorant les propriétés cosmétiques des cheveux traités par des compositions détergentes, à savoir notamment ceux décrits dans la demande de brevet EP-A-0 337 354 et dans les demandes de brevets français FR-A-2 270 846, 2 383 660, 2 598 611, 2 470 596 et 2 519 863.

De manière encore plus générale, au sens de la présente invention, l'expression "polymère cationique" désigne tout polymère contenant des groupements cationiques et/ou des groupements ionisables en groupements cationiques.

Parmi tous les polymères cationiques susceptibles d'être utilisés dans le cadre de la présente invention, on préfère mettre en oeuvre les dérivés d'éther de cellulose quaternaires tels que les produits commercialisés sous la dénomination « JR 400 » par la société UNION CARBIDE CORPORATION, les cyclopolymères, en particulier les homopolymères de sel de diallyldiméthylammonium et les copolymères de sel de diallyldiméthylammonium et d'acrylamide en particulier les chlorures, commercialisés sous les dénominations « MERQUAT 100 », « MERQUAT 550» et « MERQUAT S» par la société MERCK, les polysaccharides cationiques et plus particulièrement les gommes de guar modifiées par du chlorure de 2,3-époxypropyl triméthylammonium commercialisées par exemple sous la dénomination « JAGUAR C13S » par la société MEYHALL.

Selon l'invention, le ou les polymères cationiques peuvent représenter de 0,001 % à 10 % en poids, de préférence de 0,005 % à 5 % en poids, et encore plus préférentiellement de 0,01 % à 3 % en poids, du poids total de la composition finale.

Les compositions selon l'invention peuvent contenir également des synergistes de mousses tels que des 1,2-alcanediols en C₁₀-C₁₈ ou des alcanolamides gras dérivés de mono ou de diéthanolamine.

Bien entendu, l'homme du métier veillera à choisir ce ou ces éventuels composés complémentaires et/ou leurs quantités de manière telle que les propriétés avantageuses attachées intrinsèquement à l'association (base lavante + esters selon l'invention) conforme à l'invention ne soient pas, ou substantiellement pas, altérées par la ou les adjonctions envisagées.

Ces compositions peuvent se présenter sous la forme de liquides plus ou moins épaissis, de crèmes ou de gels et elles conviennent principalement au lavage, au soin des cheveux.

Lorsque les compositions conformes à l'invention sont mises en oeuvre comme des shampooings classiques, elles sont simplement appliquées sur cheveux mouillés et la mousse générée par massage ou friction avec les mains est ensuite éliminée, après un éventuel temps de pause, par rinçage à l'eau, l'opération pouvant être répétée une ou plusieurs fois.

L'invention a également pour objet un procédé de lavage et de conditionnement des consistant à appliquer sur lesdites matières mouillées une quantité efficace d'une composition telle que définie ci-dessus, puis à effectuer un rinçage à l'eau après un éventuel temps de pause.

Un exemple concret, mais nullement limitatif, illustrant l'invention va maintenant être donné.

### EXEMPLE 1

On a réalisé deux compositions de shampooings, l'une conforme à l'invention (composition A) et l'autre comparative (composition B):

| | A Invention | B Comparatif |
|---|---|---|
| - Lauryléthersulfate de sodium (C12/C14 à 70/30) à 2,2 moles d'oxyde d'éthylène (en solution aqueuse à 70% de MA) (MA = matière active) | 12,6 gMA | 12,6 gMA |
| - Disodium Cocoamphodiacetate (MIRANOL C2M conc. NP (*) (en solution aqueuse à 38 % de MA) | 2,7 gMA | 2,7 gMA |
| - Néopentanoate d'isostéaryle | 1 g | - |
| - Gomme de cellulose cationique (POLYMER JR 400 de UNION CARBIDE) | 0,2 g | 0,2 g |
| - Distéarate d'éthylèneglycol | 1,5 g | 1,5 g |
| - NaCl | 1,1 g | 1,1 g |
| - Cétéarylsulfate de sodium (90%MA) | 0,7 gMA | 0,7 gMA |
| - Monoisopropanolamide d'acides de coprah | 1,5 g | 1,5 g |
| - Parfum, conservateur | qs | qs |
| - Acide citrique | 0,6 g | 0,6 g |
| - Eau déminéralisée qs | 100 g | 100 g |

| | | |
|---|---|---|
| (*) MIRANOL C2M Conc. commercialisé par RHODIA CHIMIE 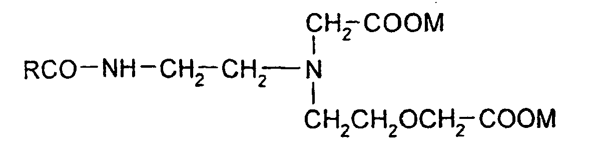 R désigne un mélange de radicaux alkyles en C₈-C₁₈ dérivé de coprah M représente Na | | |

On a effectué un shampooing en appliquant environ 12 g de la composition A sur des cheveux sensibilisés préalablement mouillés. On fait mousser le shampooing puis on rince abondamment à l'eau.
On procède selon le même mode opératoire que ci-dessus avec la composition comparative B.

Un panel d'experts a évalué les propriétés moussantes et les propriétés cosmétiques des deux compositions.

Les experts indiquent que le démarrage de la mousse est très facile et la mousse est plus aérée avec la composition A selon l'invention.

Les cheveux mouillés traités avec la composition A se démêlent plus facilement et sont plus souple. Les cheveux séchés sont plus lisses, avec plus de gonflant et de discipline.

### EXEMPLE 2

On a réalisé une composition de shampooing conforme à l'invention :

| | |
|---|---|
| - Lauryléthersulfate de sodium (C12/C14 à 70/30) à 2,2 moles d'oxyde d'éthylène (en solution aqueuse à 70% de MA) (MA = matière active) | 12,6 gMA |
| - Disodium Cocoamphodiacetate (MIRANOL C2M conc. NP (*) (en solution aqueuse à 38 % de MA) | 2,7 gMA |
| - Néopentanoate de tridécyle | 1,5 g |
| - Gomme de cellulose cationique (POLYMER JR 400 de UNION CARBIDE) | 0,2 g |
| - Distéarate d'éthylèneglycol | 1,5 g |
| - Cétéarylsulfate de sodium (90%MA) | 0,7 gMA |
| - Monoisopropanolamide d'acides de coprah | 4 g |
| - Parfum, conservateur | qs |
| - NaOH | 0,1 g |
| - Acide citrique | 0,55 g |
| - Eau déminéralisée qs | 100 g |

| | |
|---|---|
| (*) MIRANOL C2M Conc. commercialisé par RHODIA CHIMIE 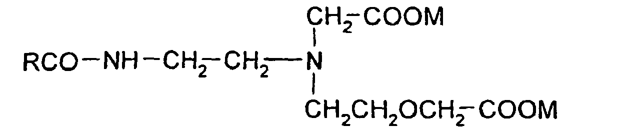 R désigne un mélange de radicaux alkyles en C₈-C₁₈ dérivé de coprah M représente Na | |

On a effectué un shampooing en appliquant environ 12 g de la composition A sur des cheveux sensibilisés préalablement mouillés. On fait mousser le shampooing puis on rince abondamment à l'eau.

Le démarrage de la mousse est très facile et la mousse est aérée.

Les cheveux mouillés traités avec la composition se démêlent facilement et sont souple. Les cheveux séchés sont lisses, avec du gonflant et de la discipline.

## Revendications

1. Composition cosmétique capillaire détergente et conditionnante,
**caractérisée par le fait qu'**elle comprend, dans un milieu aqueux cosmétiquement acceptable, (A) 4 à 50% d'une base lavante et (B) au moins un ester liquide d'acide carboxylique ayant de 4 à 6 atomes de carbone et d'alcool ayant de 12 à 26 atomes de carbone, ledit ester présentant la formule suivante:
R₁ COOR₂ (I)
dans laquelle:
R₁ désigne un radical hydrocarboné, ramifié, éventuellement mono ou polyhydroxylé, ayant de 3 à 5 atomes de carbone,
R₂ désigne un radical hydrocarboné, linéaire ou ramifié, éventuellement mono ou polyhydroxylé, ayant de 12 à 26 atomes de carbone,

2. Composition selon la revendication 1, **caractérisée par le fait que** ladite base lavante comprend un ou plusieurs tensioactifs choisis parmi des tensioactifs anioniques, amphotères, non ioniques et leurs mélanges.

3. Composition selon la revendication 1 ou 2, **caractérisée par le fait que** ladite base lavante est présente à une teneur pondérale comprise entre 6 % et 35 % en poids et plus préférentiellement comprise entre 8 % et 25 % en poids.

4. Composition selon la revendication 1, **caractérisée par le fait que** R₁ désigne un radical alkyle ramifié ayant de 3 à 5 atomes de carbone, R₂ désigne un radical alkyle ayant 12 à 26 atomes de carbone, plus particulièrement ramifié.

5. Composition selon l'une quelconque des revendications 1 à 4, **caractérisée par le fait que** R₁ désigne un radical tertio-butyle,

6. Composition selon l'une quelconque des revendications 1 à 5, **caractérisée par le fait que** R₂ est choisi parmi les radicaux tridécyle, isocétyle, isostéaryle, octyldodécyle et isoarachidyle.

7. Composition selon l'une quelconque des revendications 1 à 6, **caractérisée par le fait que** ledit ester est présent dans des concentrations comprises entre 0,1 et 20 %, et de préférence entre 0.2 et 10 % en poids par rapport au poids total de la composition.

8. Composition selon l'une quelconque des revendications 1 à 7, **caractérisée par le fait qu'**elle contient en plus un ou plusieurs adjuvants choisis par les tensioactifs cationiques, les polymères anioniques ou non ioniques ou cationiques ou amphotères, les protéines, les céramides, les pseudocéramides ou les silicones, les huiles végétales, les huiles de synthèse, les hydroxyacides, les vitamines, le panthénol et leurs mélanges.

9. Composition selon la revendication 8, **caractérisée par** le fait le polymère cationique est choisi parmi les dérivés d'éther de cellulose quaternaires, les homopolymères de sel de diallyldiméthylammonium et les copolymères de sel de diallyldiméthylammonium et d'acrylamide, les polysaccharides cationiques.

10. Composition selon l'une quelconque des revendication 8 ou 9, **caractérisée en ce que** ledit polymère cationique représente de 0.001 % à 10 % en poids, de préférence de 0,005 % à 5 % en poids, et encore plus préférentiellement de 0.01 % à 3 % en poids, du poids total de la composition.

11. Utilisation d'une composition telle définie dans l'une quelconque des revendications 1 à 10 pour le nettoyage et/ou le conditionnement des cheveux.

12. Procédé de lavage et de conditionnement des cheveux consistant a appliquer sur lesdites matières mouillées une quantité efficace d'une composition telle que définie dans l'une quelconque des revendications 1 à 10 puis à effectuer un rinçage à l'eau après un éventuel temps de pause.

## Claims

1. Detergent and conditioning cosmetic hair composition, **characterized in that** it comprises, in a cosmetically acceptable aqueous medium, (A) 4 to 50% of a washing base and (B) at least one liquid ester of a carboxylic acid containing from 4 to 6 carbon atoms and of an alcohol containing from 12 to 26 carbon atoms, the said ester having the following formula:
R₁COOR₂ (I)
in which:
R₁ denotes a branched, optionally mono- or polyhydroxylated hydrocarbon-based radical containing from 3 to 5 carbon atoms,
R₂ denotes a linear or branched, optionally mono- or polyhydroxylated hydrocarbon-based radical containing from 12 to 26 carbon atoms.

2. Composition according to Claim 1, **characterized in that** the said washing base comprises one or more surfactants chosen from anionic, amphoteric and nonionic surfactants, and mixtures thereof.

3. Composition according to Claim 1 or 2, **characterized in that** the said washing base is present in an amount by weight of between 6% and 35% by weight and more preferably between 8% and 25% by weight.

4. Composition according to Claim 1, **characterized in that** R₁ denotes a branched alkyl radical containing from 3 to 5 carbon atoms,
R₂ denotes an alkyl radical containing 12 to 26 carbon atoms, which is more particularly branched.

5. Composition according to any one of Claims 1 to 4, **characterized in that** R₁ denotes a tert-butyl radical.

6. Composition according to any one of Claims 1 to 5, **characterized in that** R₂ is chosen from tridecyl, isocetyl, isostearyl, octyldodecyl and isoarachidyl radicals.

7. Composition according to any one of Claims 1 to 6, **characterized in that** the said ester is present in concentrations of between 0.1% and 20% and preferably between 0.2% and 10% by weight relative to the total weight of the composition.

8. Composition according to any one of Claims 1 to 7, **characterized in that** it also contains one or more adjuvants chosen from cationic surfactants, anionic, nonionic, cationic or amphoteric polymers, proteins, ceramides, pseudoceramides, silicones, plant oils, synthetic oils, hydroxy acids, vitamins and panthenol, and mixtures thereof.

9. Composition according to Claim 8, **characterized in that** the cationic polymer is chosen from quaternary cellulose ether derivatives, diallyldimethylammonium salt homopolymers, copolymers of diallyldimethylammonium salt and of acrylamide, and cationic polysaccharides.

10. Composition according to either of Claims 8 and 9, **characterized in that** the said cationic polymer represents from 0.001% to 10% by weight, preferably from 0.005% to 5% by weight and even more preferably from 0.01% to 3% by weight relative to the total weight of the composition.

11. Use of a composition as defined in any one of Claims 1 to 10, for cleaning and/or conditioning the hair.

12. Process for washing and conditioning the hair, which consists in applying an effective amount of a composition as defined in any one of Claims 1 to 10 to the said wet materials, and then in rinsing them with water, after optionally leaving the composition to stand on the materials for a period of time.

## Patentansprüche

1. Reinigende und konditionierende, kosmetische Zusammensetzung für die Haarbehandlung, **dadurch gekennzeichnet, dass** sie in einem kosmetisch akzeptablen, wässrigen Medium (A) 4 bis 50 % reinigende Basisformulierung und (B) mindestens einen flüssigen Ester einer Carbonsäure mit 4 bis 6 Kohlenstoffatomen und eines Alkohols mit 12 bis 26 Kohlenstoffatomen enthält, wobei der Ester die folgende Formel aufweist:
R₁COOR₂ (I),
worin bedeuten:
R₁ eine gegebenenfalls ein- oder mehrfach hydroxylierte, verzweigte Kohlenwasserstoffgruppe mit 3 bis 5 Kohlenstoffatomen,
R₂ eine geradkettige oder verzweigte, gegebenenfalls ein- oder mehrfach hydroxylierte Kohlenwasserstoffgruppe mit 12 bis 26 Kohlenstoffatomen.

2. Zusammensetzung nach Anspruch 1, **dadurch gekennzeichnet, dass** die reinigende Basisformulierung einen oder mehrere grenzflächenaktive Stoffe enthält, die unter den anionischen, amphoteren oder nichtionischen grenzflächenaktiven Stoffen und deren Gemischen ausgewählt sind.

3. Zusammensetzung nach Anspruch 1 oder 2, **dadurch gekennzeichnet, dass** die reinigende Basisformulierung in einem Gewichtsanteil von 6 bis 35 Gew.-% und noch bevorzugter 8 bis 25 Gew.-% vorliegt.

4. Zusammensetzung nach Anspruch 1, **dadurch gekennzeichnet, dass** R₁ eine verzweigte Alkylgruppe mit 3 bis 5 Kohlenstoffatomen bedeutet und R₂ eine Alkylgruppe mit 12 bis 26 Kohlenstoffatomen ist, die insbesondere verzweigt ist.

5. Zusammensetzung nach einem der Ansprüche 1 bis 4, **dadurch gekennzeichnet, dass** R₁ die *t*-Butylgruppe ist.

6. Zusammensetzung nach einem der Ansprüche 1 bis 5, **dadurch gekennzeichnet, dass** R₂ unter den Gruppen Tridecyl, Isocetyl, Isostearyl, Octyldodecyl und Isoarachidyl ausgewählt ist.

7. Zusammensetzung nach einem der Ansprüche 1 bis 6, **dadurch gekennzeichnet, dass** der Ester in Konzentrationen von 0,1 bis 20 % und vorzugsweise 0,2 bis 10 Gew.-%, bezogen auf das Gesamtgewicht der Zusammensetzung, enthalten ist.

8. Zusammensetzung nach einem der Ansprüche 1 bis 7, **dadurch gekennzeichnet, dass** sie ferner einen oder mehrere Zusatzstoffe enthält, die unter den kationischen grenzflächenaktiven Stoffen, anionischen oder nichtionischen oder kationischen oder amphoteren Polymeren, Proteinen, Ceramiden, Pseudoceramiden oder Siliconen, pflanzlichen Ölen, synthetischen Ölen, Hydroxysäuren, Vitaminen, Panthenol und deren Gemischen ausgewählt sind.

9. Zusammensetzung nach Anspruch 8, **dadurch gekennzeichnet, dass** das kationische Polymer unter den quartären Celluloseetherderivaten, Homopolymeren von Diallyldimethylammoniumsalzen und Copolymeren von Diallyldimethylammoniumsalzen und Acrylamid und kationischen Polysacchariden ausgewählt ist.

10. Zusammensetzung nach einem der Ansprüche 8 oder 9, **dadurch gekennzeichnet, dass** das kationische Polymer 0,001 bis 10 Gew.-%, vorzugsweise 0,005 bis 5 Gew.-% und noch bevorzugter 0,01 bis 3 Gew.-% des Gesamtgewichts der Zusammensetzung ausmacht.

11. Verwendung einer Zusammensetzung nach einem der Ansprüche 1 bis 10 für die Reinigung und/oder Konditionierung der Haare.

12. Verfahren für die Reinigung und Konditionierung der Haare, das darin besteht, auf die feuchten Keratinsubstanzen eine wirksame Menge einer Zusammensetzung nach einem der Ansprüche 1 bis 10 aufzutragen und anschließend nach einer gegebenenfalls abgewarteten Einwirkzeit mit Wasser zu spülen.
